# EUROPEAN PATENT APPLICATION

(11) **EP 4 130 289 A1**
(43) Date of publication of application: **08.02.2023**
(21) Application number: 20926889.5
(22) Date of filing: 10.11.2020
(51) Int. Cl.: C12Q 1/68, C12Q 1/6869, G16B 30/20

(54) **METHOD AND PROGRAM FOR CALCULATING BASE METHYLATION LEVELS**

(30) Priority: 25.03.2020 JP 2020055116
(71) Applicant: FUJIFILM Corporation, Tokyo 106-8620 (JP)
(72) Inventor: YAMAGUCHI, Naoko, Ashigarakami-gun, Kanagawa 258-8538 (JP); WAKITA, Maiko, Ashigarakami-gun, Kanagawa 258-8538 (JP)
(74) Representative: Jeffrey, Philip Michael
(86) International application number: PCT/JP2020/041984
(87) International publication number: WO 2021/192395

(57) **Abstract**

There is provided a method of calculating a base methylation degree from DNA sequence analysis data, which is a calculation method for a base methylation degree, including correcting a read based on quality information of sequence analysis data in a case of using a co-methylation site, using a paired-end read, using a molecular barcode, or using a plurality of sequence analysis data. There is also provided a program for causing a computer to execute the calculation method for a base methylation degree.

## Description

### BACKGROUND OF THE INVENTION

### 1. Field of the Invention

The present disclosure relates to a method of calculating a base methylation degree from DNA sequence analysis data and a program.

### 2. Description of the Related Art

There is a phenomenon in which a methyl group is attached to a carbon atom of a base that constitutes DNA and the base is methylated. Base methylation is known to act as a regulating factor of gene expression and has attracted attention as useful information for elucidating the mechanism of biological phenomena or diagnosing diseases.

Although there are several methods of measuring a methylation degree in DNA, one representative method is a method using a device that reads the base sequence of nucleic acid, that is, a sequencer. For example, there is a method (that is, a bisulfite sequencing method) in which a bisulfite treatment, polymerase chain reaction (PCR), and a sequence analysis by a sequencer are combined. In a case where DNA is treated with bisulfite, unmethylated cytosine is converted to uracil, whereas methylated cytosine remains as cytosine. That is, by the bisulfite treatment, the methylation state of cytosine (which is unmethylated or methylated) is converted into the information of sequence (uracil or cytosine) at a position thereof. Next, a DNA fragment is amplified by PCR. In this process, uracil is converted to thymine. Next, the sequence of the amplification product is analyzed using a sequencer. By determining whether the base at the position to be analyzed is thymine or cytosine, it is possible to know the methylation state of cytosine at the target site in DNA.

For example, JP2007-502126A and JP2005-514035A disclose a method of detecting base methylation, which is a modification of the bisulfite sequencing method.

### SUMMARY OF THE INVENTION

According to the bisulfite sequencing method, theoretically, it is possible to quantify the methylation degree of cytosine at any position in DNA in a range of 0 to 100%. However, in reality, the accuracy of quantification is limited due to a base conversion error during the bisulfite treatment, a PCR amplification error, a reading error of a sequencer, and the like.

The embodiments of the present disclosure have been made under the above circumstances.

An object of the present disclosure is to provide a method of more accurately calculating a base methylation degree from DNA sequence analysis data and a program.

Specific means for solving the above problems include the following aspects.
<1> A calculation method for a base methylation degree, which is a method of calculating a base methylation degree at a target site on DNA having a co-methylation site, the calculation method comprising:
   acquiring sequence analysis data obtained by subjecting DNA having at least one co-methylation site to a sequence analysis using a sequencer;
   correcting a base of the co-methylation site in a read based on quality information included in the sequence analysis data; and
   calculating a base methylation degree at the target site from corrected reads.
<2> A calculation method for a base methylation degree, which is a method of calculating a base methylation degree at a target site on DNA having a co-methylation site, the calculation method comprising:
   acquiring sequence analysis data obtained by subjecting DNA having at least one co-methylation site to a sequence analysis using a sequencer;
   correcting a read based on quality information included in the sequence analysis data and excluding a read in which a base does not coincide between the co-methylation sites; and
   calculating a base methylation degree at the target site from remaining reads.
<3> A calculation method for a base methylation degree, which is a method of calculating a base methylation degree at a target site on DNA, the calculation method comprising:
   acquiring sequence analysis data obtained by subjecting DNA to a sequence analysis according to a paired-end method using a next generation sequencer;
   correcting a paired-end read based on quality information included in the sequence analysis data; and
   calculating a base methylation degree at the target site from corrected reads.
<4> A calculation method for a base methylation degree, which is a method of calculating a base methylation degree at a target site on DNA, the calculation method comprising:
   acquiring sequence analysis data obtained by subjecting DNA to a sequence analysis according to a paired-end method using a next generation sequencer;
   correcting a read based on quality information included in the sequence analysis data and excluding a paired-end read in which a base at the target site does not coincide between the paired-end reads; and
   calculating a base methylation degree at the target site from remaining reads.
<5> A calculation method for a base methylation degree, which is a method of calculating a base methylation degree at a target site on DNA, the calculation method comprising:
   acquiring sequence analysis data obtained by subjecting DNA to which a molecular barcode is attached to a sequence analysis using a sequencer;
   correcting a read based on quality information included in the sequence analysis data;
   classifying corrected reads into a group of reads having the same molecular barcode;
   determining a base that most frequently appears at the target site on each of the read groups; and
   calculating a base methylation degree at the target site from a set of the bases that most frequently appear.
<6> A calculation method for a base methylation degree, which is a method of calculating a base methylation degree at a target site on DNA, the calculation method comprising:
   acquiring sequence analysis data obtained by subjecting DNA to which a molecular barcode is attached to a sequence analysis using a sequencer;
   correcting a read based on quality information included in the sequence analysis data;
   classifying corrected reads into a group of reads having the same molecular barcode, excluding a read having no identity in a sequence of a region including the target site in each of the read groups, and obtaining a read group having the same molecular barcode and the same sequence of the region including the target site;
   determining a base at the target site in each of the read groups having the same molecular barcode and the same sequence of the region including the target site; and
   calculating a base methylation degree at the target site from a set of the determined bases.
<7> A calculation method for a base methylation degree, which is a method of calculating a base methylation degree at a target site on DNA, the calculation method comprising:
   acquiring a plurality of sequence analysis data obtained by subjecting DNA to a plurality of sequence analyses using a sequencer;
   correcting a read based on quality information included in the sequence analysis data, for each of the sequence analysis data from an individual sequence analysis, and calculating a base methylation degree at the target site from corrected reads; and
   calculating a representative value from sets of the methylation degrees in all the sequence analyses and adopting the representative value as a base methylation degree at the target site.
<8> The calculation method for a base methylation degree according to <7>, in which in a case where the sets of the methylation degrees in all the sequence analyses vary from each other or include a specifically large or small methylation degree, or in a case where the sets of the methylation degrees in all the sequence analyses vary from each other and include a specifically large or small methylation degree, the representative value and the base methylation degree at the target site are calculated to be uncalculable.
<9> A calculation method for a base methylation degree, in which the calculation method is carried out by combining two or more selected from the group consisting of the calculation method for a base methylation degree according to <1>, the calculation method for a base methylation degree according to <2>, the calculation method for a base methylation degree according to <3>, the calculation method for a base methylation degree according to <4>, the calculation method for a base methylation degree according to <5>, the calculation method for a base methylation degree according to <6>, the calculation method for a base methylation degree according to <7>, and the calculation method for a base methylation degree according to <8>.
<10> A program for causing a computer to execute the calculation method for a base methylation degree according to any one of <1> to <9>.
<10'> A computer that is operated according to a program for causing a computer to execute the calculation method for a base methylation degree according to any one of <1> to <9>.
<11> A program for causing a computer to be executed, which calculates a base methylation degree at a target site on DNA having a co-methylation site, in which the program is for executing;
   a step of acquiring sequence analysis data obtained by subjecting DNA having at least one co-methylation site to a sequence analysis using a sequencer,
   a step of correcting a base of the co-methylation site in a read based on quality information included in the sequence analysis data, and
   a step of calculating a base methylation degree at the target site from corrected reads.
<12> A program for causing a computer to be executed, which calculates a base methylation degree at a target site on DNA having a co-methylation site, in which the program is for executing;
   a step of acquiring sequence analysis data obtained by subjecting DNA having at least one co-methylation site to a sequence analysis using a sequencer,
   a step of correcting a read based on quality information included in the sequence analysis data and excluding a read in which a base does not coincide between the co-methylation sites, and
   a step of calculating a base methylation degree at the target site from remaining reads.
<13> A program for causing a computer to be executed, which calculates a base methylation degree at a target site on DNA, in which the program is for executing;
   a step of acquiring sequence analysis data obtained by subjecting DNA to a sequence analysis according to a paired-end method using a next generation sequencer,
   a step of correcting a paired-end read based on quality information included in the sequence analysis data, and
   a step of calculating a base methylation degree at the target site from corrected reads.
<14> A program for causing a computer to be executed, which calculates a base methylation degree at a target site on DNA, in which the program is for executing;
   a step of acquiring sequence analysis data obtained by subjecting DNA to a sequence analysis according to a paired-end method using a next generation sequencer,
   a step of correcting a read based on quality information included in the sequence analysis data and excluding a paired-end read in which a base at the target site does not coincide between the paired-end reads, and
   a step of calculating a base methylation degree at the target site from remaining reads.
<15> A program for causing a computer to be executed, which calculates a base methylation degree at a target site on DNA, in which the program is for executing;
   a step of acquiring sequence analysis data obtained by subjecting DNA to which a molecular barcode is attached to a sequence analysis using a sequencer,
   a step of correcting a read based on quality information included in the sequence analysis data,
   a step of classifying corrected reads into a group of reads having the same molecular barcode,
   a step of determining a base that most frequently appears at the target site on each of the read groups, and
   a step of calculating a base methylation degree at the target site from a set of the bases that most frequently appear.
<16> A program for causing a computer to be executed, which calculates a base methylation degree at a target site on DNA, in which the program is for executing;
   a step of acquiring sequence analysis data obtained by subjecting DNA to which a molecular barcode is attached to a sequence analysis using a sequencer,
   a step of correcting a read based on quality information included in the sequence analysis data,
   a step of classifying corrected reads into a group of reads having the same molecular barcode, excluding a read having no identity in a sequence of a region including the target site in each of the read groups, and obtaining a read group having the same molecular barcode and the same sequence of the region including the target site,
   a step of determining a base at the target site on each of the read groups having the same molecular barcode and the same sequence of the region including the target site, and
   a step of calculating a base methylation degree at the target site from a set of the determined bases.
<17> A program for causing a computer to be executed, which calculates a base methylation degree at a target site on DNA, in which the program is for executing;
   a step of acquiring a plurality of sequence analysis data obtained by subjecting DNA to a plurality of sequence analyses using a sequencer,
   a step of correcting a read based on quality information included in the sequence analysis data, for each of the sequence analysis data from an individual sequence analysis, and calculating a base methylation degree at the target site from corrected reads, and
   a step of calculating a representative value from sets of the methylation degrees in all the sequence analyses and adopting the representative value as a base methylation degree at the target site.
<18> The program according to <17>, in which in a case where the sets of the methylation degrees in all the sequence analyses vary from each other or include a specifically large or small methylation degree, or in a case where the sets of the methylation degrees in all the sequence analyses vary from each other and include a specifically large or small methylation degree, the representative value and the base methylation degree at the target site are calculated to be uncalculable.
<19> A program for causing a computer to be executed by combining two or more selected from the group consisting of the program according to <11>, the program according to <12>, the program according to <13>, the program according to <14>, the program according to <15>, the program according to <16>, the program according to <17>, and the program according to <18>.
<20> A computer that is operated according to the program according to any one of <11> to <19>.

According to the present disclosure, there are provided a method of more accurately calculating a base methylation degree from DNA sequence analysis data and a program.

### BRIEF DESCRIPTION OF THE DRAWINGS

Fig. 1 is a flowchart illustrating a flow of an embodiment 1-1.
Fig. 2 is a flowchart illustrating a flow of an embodiment 1-2.
Fig. 3 is a flowchart illustrating a flow of an embodiment 2-1.
Fig. 4 is a flowchart illustrating a flow of an embodiment 2-2.
Fig. 5 is a flowchart illustrating a flow of an embodiment 3-1.
Fig. 6 is a flowchart illustrating a flow of an embodiment 3-2.
Fig. 7 is a flowchart illustrating a flow of an embodiment 4-1.
Fig. 8 is a configuration diagram of the hardware of a computer.

### DESCRIPTION OF THE PREFERRED EMBODIMENTS

Hereinafter, embodiments of the present disclosure will be described. These descriptions and Examples are only illustrative of the embodiments and do not limit the scope of the embodiments.

In the present disclosure, a numerical range expressed using "to" indicates a range including numerical values before and after "to" as a minimum value and a maximum value.

The meanings of the terms that are used in this disclosure are as follows.

The target site in DNA means a position that is targeted for calculating the methylation degree according to the method and program of the present disclosure. The target site in the DNA is random.

The base methylation degree is a value calculated from a set of DNA fragments, and it is calculated for each base in DNA. A base methylation degree is {the number of DNA fragments in which a base is methylated/(the number of DNA fragments in which a base is methylated + the number of DNA fragments in which a base is unmethylated)}, and it is indicated in terms of percentage (%).

The sequence analysis data includes entire information output by a sequencer regarding the sequence analysis, such as the base sequence of each read, the identity of the sequence between the reads, and the quality information of the sequence analysis. The quality information is information including at least one of the sequence certainty of one sequence processing, the sequence certainty of each read, or the base certainty at each position.

The sequencer is a term including a first generation sequencer (a capillary sequencer), a second generation sequencer (a next generation sequencer), a third generation sequencer, a fourth generation sequencer, and a sequencer to be developed in the future. Unless otherwise specified, the sequencer may be a capillary sequencer, may be a next generation sequencer, or may be another sequencer. The sequencer is preferably a next generation sequencer from the viewpoints of the speed of analysis, the large number of samples that can be processed at one time, and the like. The next generation sequencer (NGS) refers to a sequencer that is classified by being contrasted with a capillary sequencer (called a first generation sequencer) using the Sanger method. At present, the most popular next generation sequencer is a sequencer of which the principle is to capture fluorescence or luminescence linked to a complementary strand synthesis by DNA polymerase or a complementary strand binding by DNA ligase and determine the base sequence. Specific examples thereof include MiSeq (Illumina, Inc.), HiSeq 2000 (Illumina, Inc., HiSeq is a registered trade name), and Roche 454 (Roche, Ltd.).

The read refers to a unit of a base sequence that has been subjected to a read treatment by a sequencer.

The correcting of a read is carried out based on the quality information included in the sequence analysis data. The read correction includes at least any one of the exclusion of a read in which the sequence certainty is absolutely or relatively low, the selection of a read in which the sequence certainty is absolutely or relatively high, or the correction of the individual base (for example, the replacement of a base having a high presence certainty with a base having low presence certainty).

A co-methylation site refers to two or more methylation sites in a case where the two or more methylation sites at different positions on DNA are presumed to be in the same methylation state (both methylated or both unmethylated).

The co-methylation site is, for example, two adjacent CpG sites (two base sequences in which guanine appears next to cytosine) with one or a plurality of bases being sandwiched therebetween.

The paired-end method is a method of reading a base sequence from both ends of a nucleic acid.

The paired-end read means a read pair that has been read from both ends of one base sequence.

The molecular barcode is a synthetic nucleic acid having a mutually different sequence, which is attached to distinguish a plurality of nucleic acids to be measured, from each other. In a case a unique molecular barcode is attached to a nucleic acid to be measured, before amplification, it is possible to identify the amplification product from the nucleic acid to be measured.

The present disclosure discloses a method of acquiring sequence analysis data obtained by subjecting DNA to a sequence analysis using a sequencer and calculating a base methylation degree at a target site in DNA from the sequence analysis data and a program. Examples of the base at the target site include cytosine and adenine.

For a DNA sequence analysis using a sequencer, the bisulfite sequencing method is preferable in a case where the base at the target site is cytosine. Examples of embodiments of the bisulfite sequencing method include subjecting DNA to a bisulfite treatment, carrying out PCR using a primer pair, and subjecting an amplification product to a sequence analysis using a sequencer.

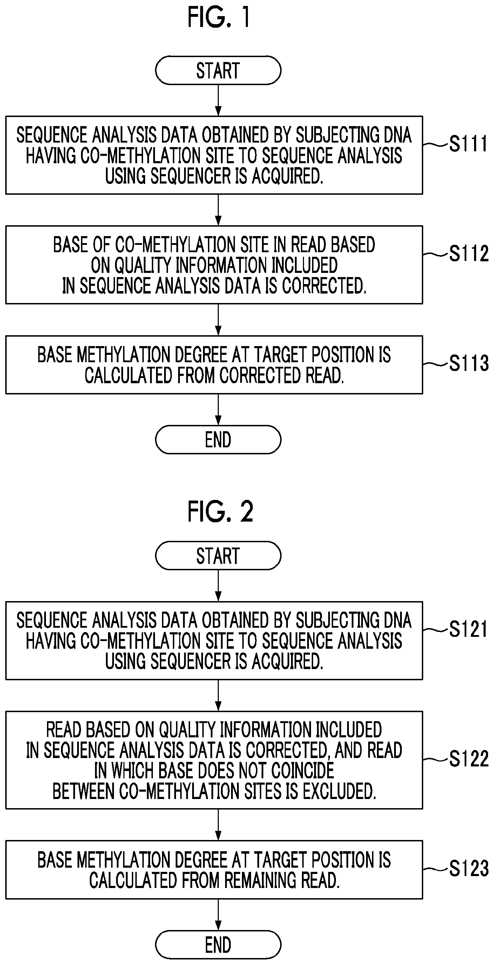

The present disclosure discloses a first embodiment, a second embodiment, a third embodiment, and a fourth embodiment, as a method of calculating a base methylation degree and a program. Hereinafter, each embodiment will be described with reference to the flowcharts illustrated in Fig. 1 to Fig. 7.

### <First embodiment: Embodiment using co-methylation site>

A first embodiment is a method of calculating a base methylation degree at a target site in DNA from sequence analysis data obtained by subjecting DNA having at least one co-methylation site to a sequence analysis. The first embodiment is an embodiment that can be carried out in a case where there is a co-methylation site in the DNA to be analyzed and the base at the target site constitutes the co-methylation site.

The co-methylation site in DNA can be identified according to a list of co-methylation sites or a search algorithm. The first embodiment may further include identifying the co-methylation site in the DNA to be analyzed according to a list of co-methylation sites or a search algorithm.

The list of co-methylation sites can be constructed by obtaining information on the methylation sites from an existing gene database. The search algorithm for a co-methylation site is, for example, an algorithm for searching for two adjacent CpG sites, with 1 or more and 10 or less of bases being sandwiched therebetween.

In a case of using the co-methylation site, the first embodiment improves the accuracy of the base methylation degree by correcting a read based on the quality information of the sequence analysis data. The first embodiment is classified into two embodiments (referred to as an embodiment 1-1 and embodiment 1-2) depending on the way how the co-methylation site is used.

### [Embodiment 1-1]

Fig. 1 is a flowchart illustrating a flow of an embodiment 1-1. The embodiment 1-1 includes a step shown as S111, a step shown as S112, and a step shown as S113.

The co-methylation site in DNA is expected to be in the same methylation state (both methylated or unmethylated). However, in a case where the C/T sequences of the co-methylation sites in the read are different from each other, it is presumed that a measurement error (for example, a base conversion error during the bisulfite treatment, a PCR amplification error, or a reading error of a sequencer) has occurred in at least one base of the co-methylation site. In the embodiment 1-1, the measurement error is corrected at the step shown as S112.

Each step will be described below.

At the step shown as S111, sequence analysis data obtained by subjecting DNA having at least one co-methylation site to a sequence analysis using a sequencer is acquired. Then, the process proceeds to the step shown as S112.

At the step shown as S112, a base of the co-methylation site in a read based on quality information included in the sequence analysis data is corrected. Specifically, it is preferable to carry out a correction by which in the co-methylation sites in the read, a base of a site where the C/T sequence reliability is low is replaced with a base of a site where the C/T sequence reliability is high. In a case where C/T sequences differ between the co-methylation sites in the read, the C/T sequences between the co-methylation sites in the read are replaced with the same sequence at the step shown as S112.

Next, at the step shown as S113, a base methylation degree at the target site is calculated from a corrected read. Since the methylation degree is calculated from a set of reads in which the base certainty at the target site is increased, the accuracy of the base methylation degree is improved.

### [Embodiment 1-2]

Fig. 2 is a flowchart illustrating a flow of an embodiment 1-2. The embodiment 1-2 includes a step shown as S121, a step shown as S122, and a step shown as S123.

The co-methylation site in DNA is expected to be in the same methylation state (both methylated or unmethylated). However, in a case where the C/T sequences of the co-methylation sites in the read are different from each other, it is presumed that a measurement error (for example, a base conversion error during the bisulfite treatment, a PCR amplification error, or a reading error of a sequencer) has occurred in at least one base of the co-methylation site. In the embodiment 1-2, the measurement error is corrected at the step shown as S122.

Each step will be described below.

At the step shown as S121, sequence analysis data obtained by subjecting DNA having at least one co-methylation site to a sequence analysis using a sequencer is acquired. Then, the process proceeds to the step shown as S122.

At the step shown as S122, a read based on quality information included in the sequence analysis data is corrected, and further, a read in which a base does not coincide between the co-methylation sites is excluded. The read correction is preferably the exclusion of a read in which the sequence certainty of the entire read or the base certainty at the target site is absolutely or relatively low, or the selection of a read in which the sequence certainty of the entire read or the base certainty at the target site is absolutely or relatively high. Next, a read in which the base does not coincide between the co-methylation sites is excluded. At the step shown as S122, the original reads are narrowed down to form a population of reads having high sequence reliability.

Next, at the step shown as S123, a base methylation degree at the target site is calculated from a remaining read. Since the methylation degree is calculated from a set of reads having high sequence reliability, the accuracy of the base methylation degree is improved.

### <Second embodiment: Embodiment using paired-end read>

A second embodiment is a method of calculating a base methylation degree at a target site in DNA from sequence analysis data obtained by subjecting DNA to a sequence analysis according to a paired-end method using a next generation sequencer. In a case of using the paired-end read, the second embodiment improves the accuracy of the base methylation degree by correcting a read based on the quality information of the sequence analysis data. The second embodiment is classified into two embodiments (referred to as an embodiment 2-1 and embodiment 2-2) depending on the way how the paired-end read is used.

### [Embodiment 2-1]

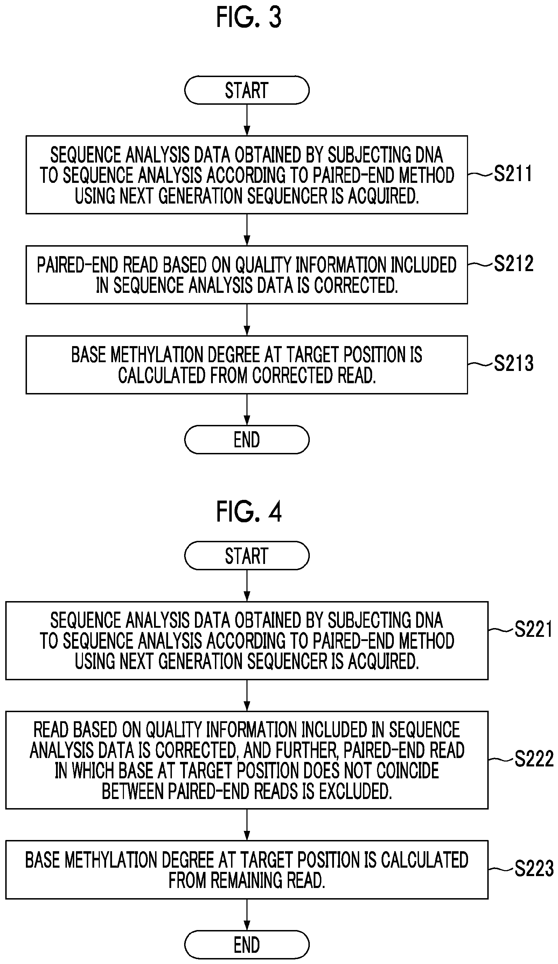

Fig. 3 is a flowchart illustrating a flow of an embodiment 2-1. The embodiment 2-1 includes a step shown as S211, a step shown as S212, and a step shown as S213.

The read pair that constitutes one paired-end read is expected to have the same sequence. However, in a case where the sequences between the paired-end reads are different from each other, it is presumed that a reading error of a sequencer has occurred in at least one read of the paired-end read. In the embodiment 2-1, the measurement error is corrected at the step shown as S212.

Each step will be described below.

At the step shown as S211, sequence analysis data obtained by subjecting DNA to a sequence analysis according to a paired-end method using a next generation sequencer is acquired. Then, the process proceeds to the step shown as S212.

At the step shown as S212, a paired-end read based on quality information included in the sequence analysis data is corrected. For the read correction, it is preferable to select a read in which a base certainty at the target site is absolutely or relatively high and use this read as a representative of the paired-end read. In a case where the sequences between the paired-end reads are different from each other, the read sequence is corrected in regard to the target site at the step shown as S212.

Next, at the step shown as S213, a base methylation degree at the target site is calculated from a corrected read. Since the methylation degree is calculated from a set of reads in which the base certainty at the target site is increased, the accuracy of the base methylation degree is improved.

### [Embodiment 2-2]

Fig. 4 is a flowchart illustrating a flow of an embodiment 2-2. The embodiment 2-2 includes a step shown as S221, a step shown as S222, and a step shown as S223.

The read pair that constitutes one paired-end read is expected to have the same sequence. However, in a case where the sequences between the paired-end reads are different from each other, it is presumed that a reading error of a sequencer has occurred in at least one read of the paired-end read. In the embodiment 2-2, the measurement error is corrected at the step shown as S222.

Each step will be described below.

At the step shown as S221, sequence analysis data obtained by subjecting DNA to a sequence analysis according to a paired-end method using a next generation sequencer is acquired. Then, the process proceeds to the step shown as S222.

At the step shown as S222, a read based on quality information included in the sequence analysis data is corrected, and further, a paired-end read in which a base at the target site does not coincide between the paired-end reads is excluded. The read correction is preferably the exclusion of a read in which the sequence certainty of the entire read or the base certainty at the target site is absolutely or relatively low, or the selection of a read in which the sequence certainty of the entire read or the base certainty at the target site is absolutely or relatively high. Next, a paired-end read in which a base at the target site does not coincide between the paired-end reads is excluded. At the step shown as S222, the original reads are narrowed down to form a population of reads having high sequence reliability.

Next, at the step shown as S223, a base methylation degree at the target site is calculated from a remaining read. Since the methylation degree is calculated from a set of reads having high sequence reliability, the accuracy of the base methylation degree is improved.

### <Third embodiment: Embodiment using molecular barcode>

A third embodiment is a method of calculating a base methylation degree at a target site in DNA from sequence analysis data obtained by subjecting DNA to which a molecular barcode is attached to a sequence analysis. In a case of using the molecular barcode, the third embodiment improves the accuracy of the base methylation degree by correcting a read based on the quality information of the sequence analysis data. The third embodiment is classified into two embodiments (referred to as an embodiment 3-1 and embodiment 3-2) depending on the way how the molecular barcode is used.

### [Embodiment 3-1]

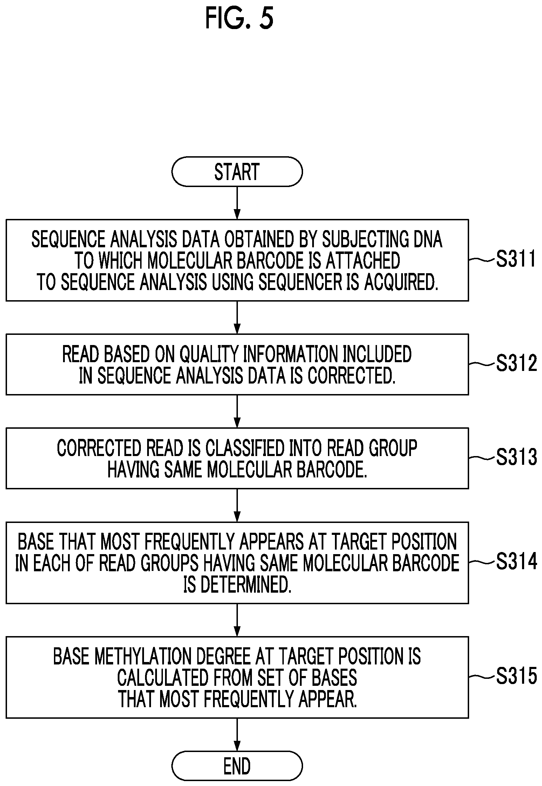

Fig. 5 is a flowchart illustrating a flow of an embodiment 3-1. The embodiment 3-1 includes a step shown as S311, a step shown as S312, a step shown as S313, a step shown as S314, and, a step shown as S315.

The read group having the same molecular barcode is expected to have the same sequence. However, in a case where this read group contains a read having a different sequence, it is presumed that a measurement error (for example, a PCR amplification error or a reading error of a sequencer) has occurred in this read. In the embodiment 3-1, since a series of the steps shown as S311 to S315 go through, the influence of the measurement error on the calculation of the base methylation degree is reduced.

Each step will be described below.

At the step shown as S311, sequence analysis data obtained by subjecting DNA to which a molecular barcode is attached to a sequence analysis using a sequencer is acquired. Then, the process proceeds to the step shown as S312.

At the step shown as S312, a read based on quality information included in the sequence analysis data is corrected. The read correction is preferably the exclusion of a read in which the sequence certainty of the entire read or the base certainty at the target site is absolutely or relatively low, or the selection of a read in which the sequence certainty of the entire read or the base certainty at the target site is absolutely or relatively high.

Next, at the step shown as S313, a corrected read is classified into a read group having the same molecular barcode. Then, the process proceeds to the step shown as S314.

At the step shown as S314, a base that most frequently appears at the target site in each of the read groups having the same molecular barcode is determined. Then, the process proceeds to the step shown as S315.

At the step shown as S315, a base methylation degree at the target site is calculated from a set of the bases that most frequently appear. Since the step shown as S311 to S315 go through, the base certainty at the target site is increased, whereby the accuracy of the base methylation degree is improved.

### [Embodiment 3-2]

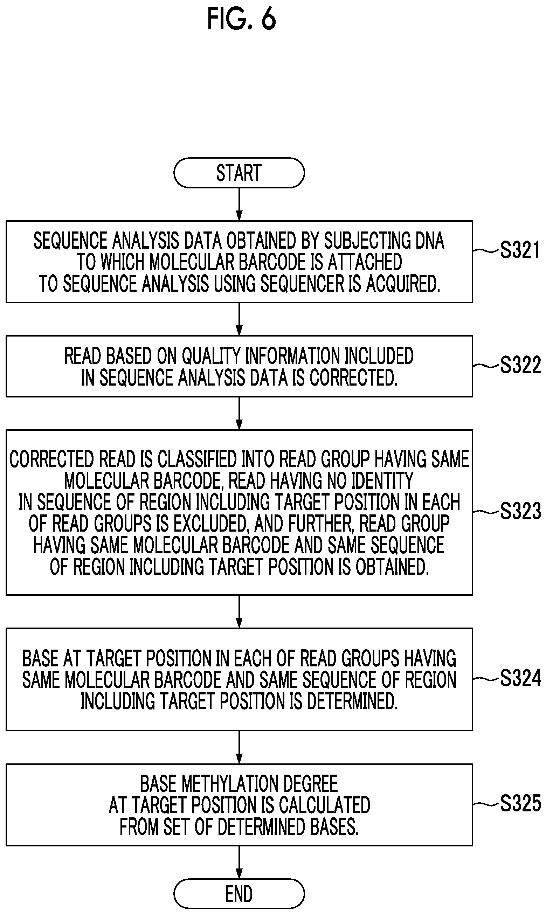

Fig. 6 is a flowchart illustrating a flow of an embodiment 3-2. The embodiment 3-2 includes a step shown as S321, a step shown as S322, a step shown as S323, a step shown as S324, and, a step shown as S325.

The read group having the same molecular barcode is expected to have the same sequence. However, in a case where this read group contains a read having a different sequence, it is presumed that a measurement error (for example, a PCR amplification error or a reading error of a sequencer) has occurred in this read. In the embodiment 3-2, since a series of the steps shown as S321 to S325 go through, the influence of the measurement error on the calculation of the base methylation degree is reduced.

Each step will be described below.

At the step shown as S321, sequence analysis data obtained by subjecting DNA to which a molecular barcode is attached to a sequence analysis using a sequencer is acquired. Then, the process proceeds to the step shown as S322.

At the step shown as S322, a read based on quality information included in the sequence analysis data is corrected. The read correction is preferably the exclusion of a read in which the sequence certainty of the entire read or the base certainty at the target site is absolutely or relatively low, or the selection of a read in which the sequence certainty of the entire read or the base certainty at the target site is absolutely or relatively high.

Next, at the step shown as S323, a corrected read is classified into a read group having the same molecular barcode, a read having no identity in a sequence of a region including the target site in each of the read groups is excluded, and further, a read group having the same molecular barcode and the same sequence of the region including the target site is obtained. Here, the region including the target site may be a part of the read or the entire length of the read. The region including the target site is preferably a region having a base length of 5 or more. Regarding the sequence identity, the information included in the sequence analysis data may be adopted, and in a case where it does not satisfy a predetermined determination criterion, it is determined that there is no sequence identity. The sequence identity is preferably 90% or more, more preferably 95% or more, and still more preferably 100%, where this numerical value may be used as a determination criterion. Sequences that satisfy a predetermined determination criterion regarding sequence identity are regarded as the same sequence.

Next, at the step shown as S324, a base at the target site in each of the read groups having the same molecular barcode and the same sequence of the region including the target site is determined. Then, the process proceeds to the step shown as S325.

At the step shown as S325, a base methylation degree at the target site is calculated from a set of the determined bases. Since the steps shown as S321 to S324 go through, the base certainty at the target site is increased, whereby the accuracy of the base methylation degree is improved.

### <Fourth Embodiment: Embodiment using plurality of sequence analysis data>

A fourth embodiment is a method of calculating a base methylation degree at a target site in DNA from a plurality of sequence analysis data obtained by subjecting DNA to a plurality of sequence analyses using a sequencer. In a case of using a plurality of sequence analysis data, the fourth embodiment improves the accuracy of the base methylation degree by correcting a read based on the quality information of the sequence analysis data.

The details of the fourth embodiment will be described in the following embodiment 4-1. Further, an embodiment 4-2 will be described as an example of the embodiment 4-1.

### [Embodiment 4-1]

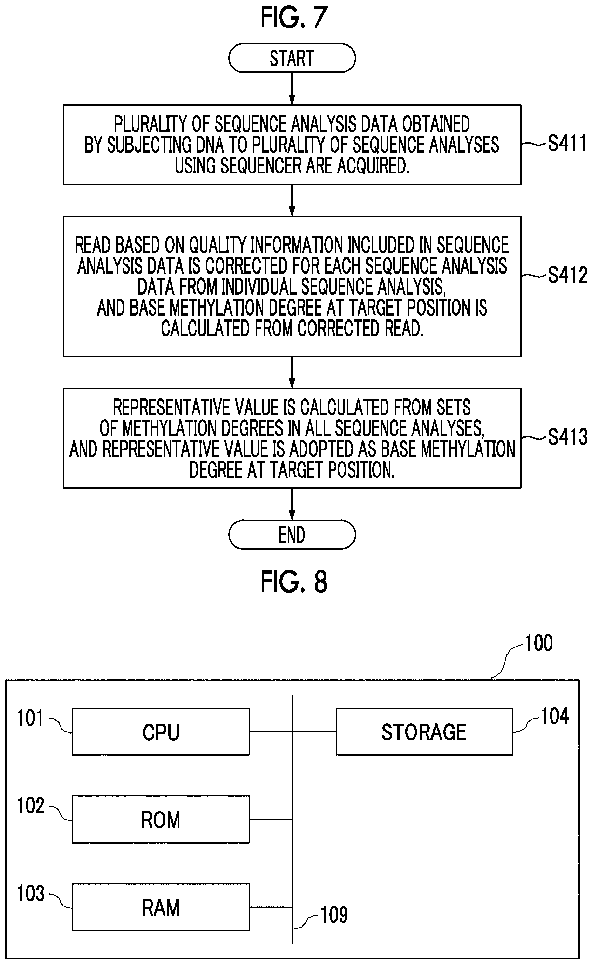

Fig. 7 is a flowchart illustrating a flow of an embodiment 4-1. The embodiment 4-1 includes a step shown as S411, a step shown as S412, and a step shown as S413.

In a case where the same DNA is used as a sample, it is ideal that the value of the base methylation degree, calculated from each of a plurality of sequence analysis data, is identical. However, it is difficult to always eliminate a measurement error of a read (for example, a base conversion error during the bisulfite treatment, a PCR amplification error, or a reading error of the sequencer), and thus the value of the base methylation degree, calculated from each of a plurality of sequence analysis data, may vary. The embodiment 4-1 is a form in which the variation in the value of the base methylation degree is excluded to improve the accuracy of the base methylation degree.

Each step will be described below.

At the step shown as S411, a plurality of sequence analysis data obtained by subjecting DNA to a plurality of sequence analyses using a sequencer are acquired. Then, the process proceeds to the step shown as S412.

At the step shown as S412, a read based on quality information included in the sequence analysis data is corrected for each of the sequence analysis data from the individual sequence analysis, and a base methylation degree at the target site is calculated from a corrected read. The read correction is preferably at least one of the exclusion of a read in which the sequence certainty of the entire read or the base certainty at the target site is absolutely or relatively low, the selection of a read in which the sequence certainty of the entire read or the base certainty at the target site is absolutely or relatively high, or the correction of the individual base.

Next, at the step shown as S413, a representative value is calculated from sets of the methylation degrees in all the sequence analyses, and the representative value is adopted as a base methylation degree at the target site. The representative value may be an average value, a median value, a mode value, or an arbitrarily defined value. Since a representative value of the base methylation degree, calculated from each of a plurality of sequence analysis data, is determined, the accuracy of the base methylation degree is improved.

### [Embodiment 4-2]

In the embodiment 4-2, at the step shown as S413 of the embodiment 4-1, in a case where the sets of the methylation degrees in all the sequence analyses vary from each other or include a specifically large or small methylation degree, or in a case where the sets of the methylation degrees in all the sequence analyses vary from each other and include a specifically large or small methylation degree, the representative value and the base methylation degree at the target site are calculated to be uncalculable. The embodiment 4-2 is a form in which a methylation degree having low reliability is output, and it is determined to be uncalculable.

In a case where at least one of the first embodiment, the second embodiment, the third embodiment, or the fourth embodiment, which are described above, are carried out, the base methylation degree can be calculated more accurately.

For the purpose of calculating a more accurate base methylation degree, two or more embodiments selected from the group consisting of the first embodiment, the second embodiment, the third embodiment, and the fourth embodiment may be combined and carried out.

The first embodiment, the second embodiment, the third embodiment, the fourth embodiment, and an embodiment of the combination thereof can be realized by causing a computer 100 to execute programs thereof.

As illustrated in the hardware configuration of Fig. 8, the computer 100 has a central processing unit (CPU) 101, a read only memory (ROM) 102, a random access memory (RAM) 103, and a storage 104. The respective configurations are communicably connected to each other via a bus 109.

The CPU 101 is a central arithmetic processing unit that executes various programs and controls each unit. That is, the CPU 101 reads a program from the ROM 102 or the storage 104, and executes the program using the RAM 103 as a work area. The CPU 101 executes a program recorded in the ROM 102 or the storage 104, controls each step, and carries out various arithmetic processes.

The ROM 102 stores various programs and various data. The RAM 103 as a work area temporarily stores a program or data. The storage 104 is composed of a hard disk drive (HDD), a solid state drive (SSD), or a flash memory, and it stores various programs including an operating system and various data. Sequence analysis data can also be stored in the storage 104.

In the computer 100, the CPU 101 in the above hardware configuration executes the programs illustrated in the flowcharts of Fig. 1 to Fig. 7, whereby a calculation method for a base methylation degree is realized.

Regarding the base methylation degree (%) that is calculated according to the embodiment of the present disclosure, the difference from the true value of the base methylation degree (%) is preferably as small as possible. The difference is preferably 0.2% or less, the difference is more preferably 0.1% or less, and the difference is particularly preferably is 0%.

### Examples

Hereinafter, embodiments of the invention will be further described with reference to Examples. However, the embodiments of the invention are not limited to these Examples.

### [Preparation of test DNA and primer pair]

As a test DNA, a synthetic DNA corresponding to 99 bases from the 12,516th base to the 12,614th base of the lambda phage DNA (SEQ ID NO: 1,5'-TTGATGGTATTGCACAGAATATGGCGGCGATGCTGACCGGCAGTGAGCAGAAC TGGCGCAGCTTCACCCGTTCCGTGCTGTCCATGATGACAGAAATTC-3') was prepared. Cytosine of the 25th base of SEQ ID NO: 1 is referred to as a site A, and cytosine of the 28th base of SEQ ID NO: 1 is referred to as a site B.

The following forward and reverse primers were prepared as a primer pair for amplifying the synthetic DNA of SEQ ID NO: 1 by PCR.
·· Forward primer: 5'-TTGATGGTATTGTATAGAATATGG-3' (SEQ ID NO: 2)
·· Reverse primer: 5'-AAATTTCTATCATCATAAACAACA-3' (SEQ ID NO: 3)

### <Example 1: Example of first embodiment>

The methylation degree of the site A of the synthetic DNA is to be calculated. At the time of DNA synthesis, the methylation degree of the site A is controlled to be 1.00%. Further, the methylation state of the site B is controlled to be the same as the methylation state of the site A. The site A and the site B were determined to be the co-methylation site according to an algorithm that regards two methylation sites of which the inter-base distance is within a 10 base as a co-methylation site.

100 ng of DNA was subjected to a bisulfite treatment. 10 ng of the recovered DNA was amplified by PCR using the primer pair described above. The sequence of the amplified DNA fragment was analyzed using a next generation sequencer. As a result of classifying reads into groups according to the kind of base (whether the base is cytosine or thymine) of the site A and the site B, the details thereof were as follows.

| | |
|---|---|
| ·· Read group 1: | |
| Site A = cytosine / site B = cytosine | 1599 reads |
| ·· Read group 2: | |
| Site A = thymine / site B = thymine | 154,620 reads |
| ·· Read group 3: | |
| Site A = cytosine / site B = thymine | 1,546 reads |
| ·· Read group 4: | |
| Site A = thymine / site B = cytosine | 1,558 reads |
| Total | 15,9323 reads |

When the methylation degree of the site A was calculated from the set of the read groups 1 to 4 described above, the calculation result was; "((the number of reads in the group 1) + (the number of reads in the group 3))/(the total number of reads) × 100 = (1,599 + 1,546)/159,323 × 100" = 1.97%.

Based on the sequence analysis data of the read group 1 to the read group 4, each of Examples 1-1 and 1-2 below was carried out.

### [Example 1-1: Example of embodiment 1-1]

Regarding a read in which a base is different between the site A and the site B, which are the co-methylation sites, it was regarded that a measurement error occurred at one of the sites, and then a correction was made to replace a base having low sequence reliability between the site A and site B with a base having high sequence reliability based on the quality information included in the sequence analysis data. By this correction, the read group 3 was corrected to the following read group 3-1 (a base of the site B was replaced with a base of the site A) or read group 3-2 (a base of the site A was replaced with a base of the site B), and the read group 4 was corrected to the following read group 4-1 (a base of the site A was replaced with a base of the site B) or read group 4-2 (a base of the site B was replaced with a base of the site A).

| | |
|---|---|
| ·· Read group 3-1: | |
| Site A = cytosine / site B = cytosine | 15 reads |
| ·· Read group 3-2: | |
| Site A = thymine / site B = thymine | 1,531 reads |
| ·· Read group 4-1: | |
| Site A = cytosine / site B = cytosine | 19 reads |
| ·· Read group 4-2: | |
| Site A = thymine / site B = thymine | 1,539 reads |

When the methylation degree of the site A was calculated from the set of the corrected reads, the calculation result was; "((the number of reads in the group 1) + (the number of reads in the group 3-1) + (the number of reads in the group 4-1))/(the total number of reads) × 100 = (1,599 + 15 + 19)/159,323 × 100" = 1.02%. By using the co-methylation site to increase the base certainty at the target site in the read, a value close to 1.00% of the true value could be obtained.

### [Example 1-2: Example of embodiment 1-2]

Based on the quality information included in the sequence analysis data, a correction was made for each read, by which a read in which the sequence reliability of the entire read was lower than the reference value was excluded. By this correction, the read group 1 to the read group 4 were corrected to the following read group 1' to read group 4'.

| | |
|---|---|
| ·· Read group 1': | |
| Site A = cytosine / site B = cytosine | 1,567 reads |
| ·· Read group 2': | |
| Site A= thymine / site B = thymine | 151,528 reads |
| ·· Read group 3': | |
| Site A = cytosine / site B = thymine | 1,469 reads |
| ·· Read group 4': | |
| Site A = thymine / site B = cytosine | 1,402 reads |
| Total | 155,966 reads |

Further, a read (that is, the read group 3' and the read group 4') in which the base is different between the site A and the site B, which are the co-methylation site, was excluded. When the methylation degree of the site A was calculated from the set of the remaining reads (that is, the read group 1' and the read group 2'), the calculation result was; "(the number of reads in the group 1')/((the number of reads in the group 1') + (the number of reads in the group 2')) × 100 = 1,567/(1,567 + 151,528) × 100" = 1.02%. By narrowing down the original reads to reads having high sequence reliability, a value close to 1.00% of the true value could be obtained.

### <Example 2: Example of second embodiment>

The methylation degree of the site A of the synthetic DNA is to be calculated. At the time of DNA synthesis, the methylation degree of the site A is controlled to be 1.00%.

100 ng of DNA was subjected to a bisulfite treatment. 10 ng of the recovered DNA was amplified by PCR using the primer pair described above. The sequence of the amplified DNA fragment was analyzed by the paired-end method using a next generation sequencer. One read of the paired-end read is referred to R1, and the other read thereof is referred to R2. As a result of classifying combinations of R1 and R2 into groups according to the kind of base (whether the base is cytosine or thymine) of the site A, the details thereof were as follows.

| | |
|---|---|
| ·· Paired-end read group 5: | |
| R1 = cytosine / R2 = cytosine | 1,547 pairs |
| ·· Paired-end read group 6: | |
| R1 = thymine / R2 = thymine | 153,182 pairs |
| ·· Paired-end read group 7: | |
| R1 = cytosine / R2 = thymine | 754 pairs |
| ·· Paired-end read group 8: | |
| R1 = thymine / R2 = cytosine | 808 pairs |
| Total | 156,291 pairs |

When the methylation degree of the site A was calculated from the sum of sets of the bases of R1 and the bases of R2 in the above paired-end read group 5 to the paired-end read group 8, the calculation result was; ((the numbers of pairs in the group 5) × 2 + (the number of pairs in the group 7) + (the number of pairs in the group 8))/(the number of total pairs × 2) × 100 = (1,547 × 2 + 754 + 808)/(156,291 × 2) × 100 = 1.49%.

Based on the sequence analysis data of the paired-end read group 5 to the paired-end read group 8, each of Examples 2-1 and 2-2 below was carried out.

### [Example 2-1: Example of embodiment 2-1]

Regarding a paired-end read in which a base at the site A does not coincide between the paired-end reads, it was regarded that a reading error occurred at one of the reads, and then a correction was made to select a base having high sequence reliability in regard to the site A as a representative of the paired-end read based on the quality information included in the sequence analysis data. By this correction, the paired-end read group 7 was corrected to the following read group 7-1 (selected as a representative of R1) and the read group 7-2 (selected as a representative of R2), and the paired-end read group 8 was corrected to the following read group 8-1 (selected as a representative of R2) and read group 8-2 (selected as a representative of R1). The reads that represent the paired-end read group 5 and the pair-end read group 6 are respectively shown below as the read group 5-1 and the read group 6-1.

| | |
|---|---|
| ·· Read group 5-1: Site A = cytosine | 1,547 reads |
| ·· Read group 6-1: Site A = thymine | 153,182 reads |
| ·· Read group 7-1: Site A = cytosine | 155 reads |
| ·· Read group 7-2: Site A = thymine | 599 reads |
| ·· Read group 8-1: Site A = cytosine | 165 reads |
| ·· Read group 8-2: Site A = thymine | 643 reads |
| Total | 156,291 reads |

When the methylation degree of the site A was calculated from the set of the above reads, the calculation result was; "((the number of reads in the group 5-1) + (the number of reads in the group 7-1) + (the number of reads in the group 8-1))/(the total number of reads) × 100 = (1,547 + 155 + 165)/156,291 × 100" = 1.19%. By using the paired-end read to increase the base certainty at the target site in the read, a value close to 1.00% of the true value could be obtained.

### [Example 2-2: Example of embodiment 2-2]

Based on the quality information included in the sequence analysis data, a correction was made for each read, by which a read in which the sequence reliability of the entire read was lower than the reference value was excluded. By this correction, the paired-end read group 5 to the paired-end read group 8 were corrected to the following paired-end read group 5' to paired-end read group 8'.

| | |
|---|---|
| ·· Paired-end read group 5': | |
| R1 = cytosine / R2 = cytosine | 1,516 pairs |
| ·· Paired-end read group 6': | |
| R1 = thymine / R2 = thymine | 150,118 pairs |
| ·· Paired-end read group 7': | |
| R1 = cytosine / R2 = thymine | 716 pairs |
| ·· Paired-end read group 8': | |
| R1 = thymine / R2 = cytosine | 727 pairs |
| Total | 153,077 pairs |

Further, paired-end read groups (that is, a paired-end read group 7' and a paired-end read group 8') in which a base at the site A does not coincide between the paired-end reads were excluded. When the methylation degree of the site A was calculated from the set of the remaining paired-end read groups (that is, the paired-end read group 5' and the paired-end read group 6'), the calculation result was; "(the number of pairs in the group 5')/((the number of pairs in the group 5') + (the number of pairs in the group 6')) × 100 = 1,516/(1,516 + 150,118) × 100" = 1.00%. By narrowing down the original reads to reads having high sequence reliability, a value close to 1.00% of the true value could be obtained.

### <Example 3: Example of third embodiment>

The methylation degree of the site A of the synthetic DNA is to be calculated. At the time of DNA synthesis, the methylation degree of the site A is controlled to be 1.00%.

100 ng of DNA was subjected to a bisulfite treatment. A molecular barcode in which 10 bases of adenine, guanine, cytosine, and thymine were randomly arranged was attached to 10 ng of the recovered DNA, and the DNA was amplified by PCR using a random primer. The sequence of the amplified DNA fragment was analyzed using a next generation sequencer.

Here, the methylation degree in a case where the methylation degree was calculated from the bases of the site A of all reads was; (the number of cytosines)/((the number of cytosines) + the number of thymines)) × 100 = 184,496/13,369,344 × 100 = 1.38%.

Based on the sequence analysis data of the above reads, each of Examples 3-1 and 3-2 below was carried out.

### [Example 3-1: Example of embodiment 3-1]

Based on the quality information included in the sequence analysis data, a correction was made for each read, by which a read in which the sequence reliability of the entire read was lower than the reference value was excluded. 1,310,720 reads were removed.

Next, the remaining reads were classified into read groups having the same molecular barcode, and the most frequent base of the site A was determined in each of the read groups having the same molecular barcode.

For example, in a read group in which the molecular barcode sequence is 5'-ATGATCGATC-3' (SEQ ID NO: 4), the details of the base of the site A was as follows. The most frequent base of the site A in this read group was cytosine.

| | |
|---|---|
| ·· Cytosine | 6,853 read |
| ·· Thymine | 52 reads |
| ·· Adenine | 32 reads |
| ·· Guanine | 44 reads |

For example, in a read group in which the molecular barcode sequence is 5'-CTGATCCAAT-3' (SEQ ID NO: 5), the details of the base of the site A was as follows. The most frequent base of the site A in this read group was thymine.

| | |
|---|---|
| ·· Cytosine | 43 reads |
| ·· Thymine | 8,652 read |
| ·· Adenine | 5 reads |
| ·· Guanine | 21 reads |

As a result of determining, as described above, the most frequent base of the site A in each of the read groups having the same molecular barcode, there were 2,700 groups in which the most frequent base was cytosine, and there were 259,444 groups in which the most frequent base was thymine. When the methylation degree was calculated from the set of the most frequent bases of the site A, the calculation result was; 2,700/(2,700 + 259,444) × 100 = 1.03%. By correcting a read based on quality information included in the sequence analysis data and further, reducing the influence of the measurement error on the calculation of the base methylation degree by using the molecular barcode, a value close to 1.00% of the true value could be obtained.

### [Example 3-2: Example of embodiment 3-2]

Based on the quality information included in the sequence analysis data, a correction was made for each read, by which a read in which the sequence reliability of the entire read was lower than the reference value was excluded. 1,310,720 reads were removed.

Next, the remaining reads were classified into read groups having the same molecular barcode, and further, a read having no identity in a sequence of a region including the site A in each of the read groups was excluded.

For example, in a read group (total 6981 reads) in which the molecular barcode sequence was 5'-ATGATCGATC-3' (SEQ ID NO: 4), the most frequent sequence of the sequence excluding the molecular barcode sequence was 5'-TTGATGGTATTGTATAGAATATGGCGGCGATGTTGATCGGTAGTGAGTAGAATTGG CGTAGTTTTATTCGTTTCGTGTTGTTTATGATGATAGAAATTT-3' (SEQ ID NO: 6), and in a case where reads that are not identical to this most frequent sequence (in this Example, the exact coincidence of the sequence of the entire read was considered as being identical) was excluded, the rest were 5,724 reads. The base of the site A of these 5,724 reads was cytosine.

As a result of determining, as described above, the base of the site A in each of the read groups having the same molecular barcode and having the same sequence, there were 2,673 groups in which the most frequent base was cytosine, and there were 259,471 groups in which the most frequent base was thymine. When the methylation degree was calculated from the set of the bases of the site A, the calculation result was; 2,673/(2,673 + 259,471) × 100 = 1.02%. By correcting a read based on quality information included in the sequence analysis data and further, reducing the influence of the measurement error on the calculation of the base methylation degree by using the molecular barcode, a value close to 1.00% of the true value could be obtained.

### <Example 4: Example of fourth embodiment>

The methylation degree of the site A or the methylation degree of the site B of the synthetic DNA is to be calculated. At the time of DNA synthesis, the methylation degrees are each independently controlled so that the methylation degree of the site A is 1.00%, and the methylation degree of the site B is 1.00%.

The DNA was divided into three parts to obtain a sample 1, a sample 2, and a sample 3.

100 ng of each sample of DNA was subjected to a bisulfite treatment. 10 ng of each recovered DNA was amplified by PCR using the primer pair described above. The sequence of the amplified DNA fragment was analyzed using a next generation sequencer.

Based on the three sequence analysis data, each of Examples 4-1 and 4-2 below was carried out.

### [Example 4-1: Example of embodiment 4-1]

Based on the quality information included in the sequence analysis data, a correction was made for individual sequence analysis data from the individual sample, by which a read in which the base reliability of the site A was lower than the reference value was excluded. The 1,736 reads were removed in the sample 1, the 1,803 reads were removed in the sample 2, and the 1,781 reads were removed in the sample 3.

When the methylation degree of the site A was calculated from the set of the remaining reads for each sample, it was 1.14% in the sample 1, 0.79% in the sample 2, and 1.45% in the sample 3. The calculated value taken as the methylation degree of the site A was 1.14%, which is the median value of the above three values.

### [Example 4-2: Example of embodiment 4-2]

Based on the quality information included in the sequence analysis data, a correction was made for each sequence analysis data from the individual sample, by which a read in which the base reliability of the site B was lower than the reference value was excluded. The 1,632 reads were removed in the sample 1, the 1,338 reads were removed in the sample 2, and the 1,305 reads were removed in the sample 3.

When the methylation degree of the site B was calculated from the set of the remaining reads for each sample, it was 1.25% in the sample 1, 5.32% in the sample 2, and 1.32% in the sample 3. In a case where there was a deviation of 3% or more in the methylation degree in a plurality of measurements, it was regarded that the measurement had no robustness, and the methylation degree of the site B was regarded as being uncalculable.

The method of calculating a base methylation degree and the program, disclosed in the present disclosure, are useful as research means for nucleic acid methylation in academic fields such as embryology, pathophysiology, neuroscience, and regenerative medicine.

The method of calculating a base methylation degree and the program, disclosed in the present disclosure, are useful as detection means for aberrant methylation of genes associated with diseases. The aberrant gene methylation detected by the method of calculating a base methylation degree and the program, disclosed in the present disclosure, are useful as information to assist doctor's diagnosis, a ground for a doctor to determine the necessity of detailed examinations (for example, an imaging examination), a grounds for a doctor to select a treatment method or a therapeutic drug, determination of a therapeutic effect, prognosis prediction for a patient, and the like.

The disclosure of JP2020-055116 filed on March 25, 2020, is incorporated in the present specification by reference in its entirety.

All publications, patent applications, and technical standards mentioned in the present specification are herein incorporated by reference to the same extent as in a case where each individual publication, patent application, and technical standard were specifically and individually indicated to be incorporated by reference.

### [Sequence list]

International application based on the International Patent Cooperation Treaty 20F00289W1JP20041984_0.app

## Claims

1. A calculation method for a base methylation degree, which is a method of calculating a base methylation degree at a target site on DNA having a co-methylation site, the calculation method comprising:
acquiring sequence analysis data obtained by subjecting DNA having at least one co-methylation site to a sequence analysis using a sequencer;
correcting a base of the co-methylation site in a read based on quality information included in the sequence analysis data; and
calculating a base methylation degree at the target site from corrected reads.

2. A calculation method for a base methylation degree, which is a method of calculating a base methylation degree at a target site on DNA having a co-methylation site, the calculation method comprising:
acquiring sequence analysis data obtained by subjecting DNA having at least one co-methylation site to a sequence analysis using a sequencer;
correcting a read based on quality information included in the sequence analysis data and excluding a read in which a base does not coincide between the co-methylation sites; and
calculating a base methylation degree at the target site from remaining reads.

3. A calculation method for a base methylation degree, which is a method of calculating a base methylation degree at a target site on DNA, the calculation method comprising:
acquiring sequence analysis data obtained by subjecting DNA to a sequence analysis according to a paired-end method using a next generation sequencer;
correcting a paired-end read based on quality information included in the sequence analysis data; and
calculating a base methylation degree at the target site from corrected reads.

4. A calculation method for a base methylation degree, which is a method of calculating a base methylation degree at a target site on DNA, the calculation method comprising:
acquiring sequence analysis data obtained by subjecting DNA to a sequence analysis according to a paired-end method using a next generation sequencer;
correcting a read based on quality information included in the sequence analysis data and excluding a paired-end read in which a base at the target site does not coincide between the paired-end reads; and
calculating a base methylation degree at the target site from remaining reads.

5. A calculation method for a base methylation degree, which is a method of calculating a base methylation degree at a target site on DNA, the calculation method comprising:
acquiring sequence analysis data obtained by subjecting DNA to which a molecular barcode is attached to a sequence analysis using a sequencer;
correcting a read based on quality information included in the sequence analysis data;
classifying corrected reads into a group of reads having the same molecular barcode;
determining a base that most frequently appears at the target site on each of the read groups; and
calculating a base methylation degree at the target site from a set of the bases that most frequently appear.

6. A calculation method for a base methylation degree, which is a method of calculating a base methylation degree at a target site on DNA, the calculation method comprising:
acquiring sequence analysis data obtained by subjecting DNA to which a molecular barcode is attached to a sequence analysis using a sequencer;
correcting a read based on quality information included in the sequence analysis data;
classifying corrected reads into a group of reads having the same molecular barcode, excluding a read having no identity in a sequence of a region including the target site in each of the read groups, and obtaining a read group having the same molecular barcode and the same sequence of the region including the target site;
determining a base at the target site in each of the read groups having the same molecular barcode and the same sequence of the region including the target site; and
calculating a base methylation degree at the target site from a set of the determined bases.

7. A calculation method for a base methylation degree, which is a method of calculating a base methylation degree at a target site on DNA, the calculation method comprising:
acquiring a plurality of sequence analysis data obtained by subjecting DNA to a plurality of sequence analyses using a sequencer;
correcting a read based on quality information included in the sequence analysis data, for each of the sequence analysis data from an individual sequence analysis, and calculating a base methylation degree at the target site from corrected reads; and
calculating a representative value from sets of the methylation degrees in all the sequence analyses and adopting the representative value as a base methylation degree at the target site.

8. The calculation method for a base methylation degree according to claim 7,
wherein in a case where the sets of the methylation degrees in all the sequence analyses vary from each other or include a specifically large or small methylation degree, or in a case where the sets of the methylation degrees in all the sequence analyses vary from each other and include a specifically large or small methylation degree, the representative value and the base methylation degree at the target site are calculated to be uncalculable.

9. A calculation method for a base methylation degree,
wherein the calculation method is carried out by combining two or more selected from the group consisting of the calculation method for a base methylation degree according to claim 1, the calculation method for a base methylation degree according to claim 2, the calculation method for a base methylation degree according to claim 3, the calculation method for a base methylation degree according to claim 4, the calculation method for a base methylation degree according to claim 5, the calculation method for a base methylation degree according to claim 6, the calculation method for a base methylation degree according to claim 7, and the calculation method for a base methylation degree according to claim 8.

10. A program for causing a computer to execute the calculation method for a base methylation degree according to any one of claims 1 to 9.
